(19) Europäisches Patentamt
European Patent Office
Office européen des brevets

(11) Publication number: 0 518 295 A2

(12) # EUROPEAN PATENT APPLICATION

(21) Application number: 92109757.2

(22) Date of filing: 10.06.92

(51) Int. Cl.$^5$: C07K 1/06, C07C 271/22

(30) Priority: 14.06.91 US 715297

(43) Date of publication of application: 16.12.92 Bulletin 92/51

(84) Designated Contracting States: DE FR GB

(71) Applicant: MILLIPORE CORPORATION
80 Ashby Road
Bedford Massachusetts 01730(US)

(72) Inventor: Hudson, Derek
52 El Cerrito Avenue
San Anselmo, CA 94960(US)
Inventor: Lyttle, Matthew H.
251 B Street
Pt. Reyer Sta., CA 94956(US)

(74) Representative: Henkel, Feiler, Hänzel & Partner
Möhlstrasse 37
W-8000 München 80(DE)

(54) **Allyl side chain protection in peptide synthesis.**

(57) A system for the protection of amino acid side-chain functional moieties during peptide synthesis, comprising the use of allyl protecting groups, such as, for example, allyl ether, allyl ester, allyl thioether allyloxycarbonyl and allyloxymethyl groups. The present allyl protecting groups do not exert adverse steric effects on peptide bond condensation, and are fully orthogonal to a variety of popular αN-protecting groups, including Fmoc and *t*Boc. Moreover, allyl deprotection is mild, and does not result in the formation of reactive carbonium ions. Allyl side-chain protection can be used alone or in conjunction with other types of side-chain protecting groups.

EP 0 518 295 A2

## BACKGROUND

Methods for the chemical synthesis of peptides are based upon the condensation of amino acid derivatives which have been blocked in such a manner that only the desired reaction proceeds. Generally, peptides are constructed by carrying out sequential cycles of peptide bond condensation, starting from the C-terminal amino acid. A suitable $^{\alpha}$N- and side chain-protected derivative of the C-terminal amino acid can be attached to an insoluble resin or support via the $^{\alpha}$C-moiety (solid-phase peptide synthesis, or SPPS). The $^{\alpha}$N-protecting moiety can be removed (deprotected), and the bound amino acid derivative can be reacted sequentially with $^{\alpha}$N- and side chain-protected amino acid derivatives, with $^{\alpha}$N-deprotection after each condensation (coupling) reaction. Peptide chain elongation can be similarly conducted in solution, without a solid support or resin. Upon completion of the requisite number of condensation cycles, the peptide is released from the solid support, or in the case of solution phase synthesis, is $^{\alpha}$C-deprotected, and recovered from solution. See generally, M. Bodanszky, Peptide Chemistry: A Practical Textbook, Springer-Verlag (1988), RSPN No. 0-387-18984-X; available from Sigma Chemical Co.

The choice of protecting group for the $^{\alpha}$N- and side chain functional moieties of each amino acid derivative is an important element in planning the construction of a particular peptide sequence. Following each round of peptide bond condensation, the $^{\alpha}$N-protecting group must be susceptible of being removed under conditions which do not alter the integrity of any amino acid residues in the nascent peptide chain or deprotect any protected side-chain moieties therein. Similarly, once the peptide chain has been assembled, the side-chain protecting groups must be removed without altering or impairing the integrity of the amino acid residues.

Frequently, it is necessary to use several different types of protecting groups in the synthesis of a particular peptide, in order to selectively deblock only the functional groups of interest under a specified set of conditions, at which the other types of protecting groups remain stable. For example, if $^{\alpha}$N-deblocking has been selected to proceed under acidic conditions, side-chain functional group deprotection must be stable to acid yet still smoothly cleavable under other conditions, such as in the presence of a catalyst at neutrality. The use of multiple types of protecting groups, susceptible to elimination under mutually distinct chemical conditions, in the synthesis of a single peptide, is termed "orthogonal protection". See G. Barany et al. (1987) Int. J. Pept. Prot. Res. **30:**705-739, at 709-710.

A variety of $^{\alpha}$N-protecting groups, capable of being removed under different chemical conditions, have been described; some representative protecting groups are listed in Table 1. Several of these are commercially available.

Table 1

| Representative $^{\alpha}$N-protecting groups | | |
|---|---|---|
| Chemical Name | Abbreviated as | Cleaved by |
| *tert*-butyloxycarbonyl- | *t*Boc- | $Hg^{2+}$ in TFA |
| benzyloxycarbonyl- | Z- | hydrogenolysis |
| $\alpha,\alpha'$-dimethyl-3,5-dimethoxy-benzlyoxycarbonyl- | Ddz- | acidolysis |
| 9-fluorenylmethyloxycarbonyl- | Fmoc- | piperidine/DMF |
| dithiasuccinoyl- | Dts- | thiolysis (RSH) |

In contrast to the wide choice of $^{\alpha}$N-protecting groups, only two general types of side-chain functional moiety protecting groups have been described. The first of these is based upon the benzyl group, which is removed (deblocked or deprotected) by catalytic hydrogenation, reduction, or strong acid. The second type is based upon the *tert*-butyl or triphenylmethyl group, which is susceptible to removal in the presence of trifluoroacetic acid (TFA). These groups are incompatible with some amino acids, such as arginine. Frequently, a substituted phenyl-sulfonyl group has been used for the protection of this amino acid; this type of group can be removed only under acidic or reducing conditions.

Numerous and recurrent problems have been encountered by those seeking to construct peptides using the currently-available types of side chain functional moiety protecting groups. For example, removal of the protecting group is frequently incomplete. Treatment with hydrofluoric acid (HF) will typically fail to completely remove *para*-toluenesulfonyl (Tos) groups from arginine residues, or to remove 4-methylbenzyl groups from cysteine residues. Similarly, treatment with TFA will often result in incomplete cleavage of threonine-*tert*-butyl ether or of [illegible]N-4-methoxy-2,3,6-trimethylbenzene-sulfonyl-arginine.

Furthermore, the elimination product of the deprotection step can often participate in undesired side-reactions. For example, tryptophan, tyrosine, methionine and cysteine residues are susceptible to alkylation by carbonium ions generated by acidolytic treatment of the side-chain protecting groups. G. Barany et al. (1987) Int. J. Pept. Prot. Res. **30:**705-739 at 727-730, discusses several residue-specific undesirable side-reactions.

Steric hinderance by bulky side chain protecting groups can also significantly retard or impair peptide bond coupling, particularly where the bulky group is present on the amino acid derivative being coupled or on the N-terminal residue of the peptide under construction. This effect is particularly marked with the triphenylmethyl (trityl) derivatives of asparagine, glutamine, histidine, and cysteine.

Moreover, current methods for recovering the assembled peptide from the solid support, and for conducting final deprotection of side chain functional moieties, as well as for inducing the desired final conformation in the peptide, have not proven amenable to automation or scale-up. These and other considerations serve to demonstrate that a system of side chain functional moiety protection which is non-bulky, readily removed without generating an improperly reactive elimination product, and can be adapted to automated, commercial-scale peptide synthesis, would be of great interest to practitioners in the art.

## SUMMARY OF THE INVENTION

The present invention relates to amino acid derivatives for peptide synthesis, in which the side chains thereof are protected with allyl side-chain protecting groups. More specifically, the allyl side-chain protecting groups in the present amino acid derivatives are selected from the group consisting of allyloxycarbonyl, allyloxymethyl, allyl ether, allyl thioether, allyl ester, and allyl amide. The present amino acid derivatives have the general formula:

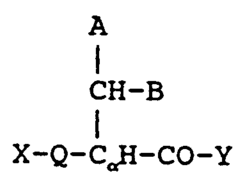

wherein the alpha carbon is either of the L or D configuration; Y is selected from the group consisting of halogens, hydroxyl groups, and active esters; X is a carbonyl-type protecting group; Q is an $\alpha$ amino group, in either di- or tri-substututed form; B is a hydrogen or a methyl group; and A is an amino acid side-chain protected by one of the above-listed allyl side-chain protecting groups.

This invention also relates to a method of producing a side-chain protected amino acid derivative for peptide synthesis. In the present method, an allyl side chain protecting group of the present invention is attached to the side-chain of the amino acid. An $^{\alpha}$N-protecting group is also attached; suitable $^{\alpha}$N-protecting groups are those which are orthogonal to the allyl side-chain protecting group. Carbonyl protecting groups, such as *tert*-butyloxycarbonyl (*t*Boc), 9-fluorenylmethyloxycarbonyl (Fmoc), and the like, are particularly suitable.

This invention further relates to a solid-phase method of peptide synthesis, in which the amino acid derivatives of the present invention are used to construct the desired peptide sequence. When the support-bound peptide has been assembled, the allyl side-chain protecting groups are removed by, for example, treating the support-bound peptide with an organometallic catalyst, such as *tetrakis*-triphenylphosphine palladium(0). The support-bound, deprotected peptide is thereafter treated with an agent sufficient to release it from the solid suppport.

The present invention relates still further to a solid-phase method of producing a cyclic peptide. In this method, the desired peptide is constructed on a solid support in the manner described above, except that allyl protection is used only for the amino acid residues which are to be linked to each other to form a circularized structure. For the other amino acid derivatives used in constructing the sequence, side-chain protecting groups which are orthogonal to the allyl groups are used. The support-bound, assembled peptide is allyl-deprotected and subjected to treatment with a cyclizing reagent, including for example, benzotriazolyl *N*-oxytrisdimethyamino-phosphonium hexafluorophosphate (BOP reagent), which induces glutamic acid-to-lysine side-chain to side-chain coupling. The resulting circular peptide is then fully deprotected and released from the solid support.

A preferred type of solid support for use in the present method of peptide synthesis is a polystyrene-graft copolymer resin. Polyethylene glycol-polystyrene graft copolymer resins are particularly preferred. Suitable resins are described in U.S.S.N. 07/576,634, filed August 31, 1990, and in U.S.S.N. 07/715,289 [Attorney's Docket No. MIL90-04A, filed June 14, 1991 , the teachings of which are incorporated herein by reference.

The present invention provides a system of side chain functional moiety protection for amino acid derivatives for peptide synthesis based upon the use of allyl groups. Allyl groups are non-bulky and therefore do not exert adverse steric influences on peptide bond condensation at sites adjacent to the protected amino acid in the peptide chain during construction. Allyl groups are smoothly cleaved under mild conditions without generating an improperly reactive elimination product such as a carbonium ion. In addition, the allyl side chain protecting system of the present invention is fully orthogonal to widely-used αN-protecting carbonyl-type groups such as *tert*-butyloxycarbonyl (*t*Boc), or 9-fluorenylmethyloxycarbonyl (Fmoc). Moreover, allyl side-chain protection can be readily adapted for automated, commercial-scale peptide synthesis.

## DETAILED DESCRIPTION OF THE INVENTION

The allyl side-chain protected amino acid derivatives of the present invention have the general formula:

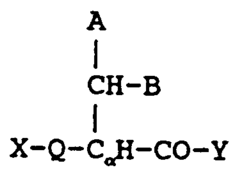

wherein the alpha carbon is either of the L or D configuration; Y is selected from the group consisting of halogens (including fluorine, chlorine, bromine, and iodine), hydroxyl groups, and active esters; X is a carbonyl-type protecting group; Q is an α amino group, in either di- or tri-substituted form; B is a hydrogen or a methyl group; and A is an amino acid side-chain protected by an allyl protecting group selected from the group consisting of allyloxycarbonyl, allyloxymethyl, allyl ether, allyl thioether, allyl ester, and allyl amide.

The term "active ester" refers to compounds which activate the carboxyl group of an amino acid or peptide. Active esters activate the carboxyl group by making it more reactive with an amino group. Active esters which can be used in the composition and methods described herein include, for example, pentafluorophenyl, nitrophenylpyrazol, benzotrialzolyloxy, and active intermediates generated by coupling agents, including O-acylisourea and symmetrical anhydrides. Active esters suitable for use with the instant invention are more fully described in U.S.S.N. 07/362,980, filed June 8, 1989, the teachings of which are incorporated herein by reference.

The $^{\alpha}$N moiety of the present composition is protected by a carbonyl-type protecting group. Suitable protecting groups include, for example, *tert*-butyloxycarbonyl (*t*Boc), *tert*-amyloxycarbonyl, adamantanyloxycarbonyl, 9-fluorenylmethyloxycarbonyl (Fmoc), 2-(3,5-dimethoxyphenyl)-2-propyloxycarbonyl, dithiasuccinoyl, biphenylylisopropyloxycarbonyl, and phenylisopropyloxycarbonyl. Such groups are susceptible to removal (deprotection) under conditions to which the allyl side-chain protecting groups are stable (i.e., orthogonal). Thus, the $^{\alpha}$N-protecting groups can be removed by, for example, subjecting the amino acid derivative to treatment with a moderate base (e.g., piperidine in DMF, which removes Fmoc), or a moderate acid (e.g., TFA, which removes *t*Boc). Fmoc and *t*Boc amino acid derivatives of the present invention are particularly useful for solid-phase peptide synthesis.

The allyl protecting groups of the present invention can be used to provide orthogonal protection to nonpolar, polar, negatively-charged, or positively-charged amino acid side chain functional moieties. More specifically, an allyloxycarbonyl group can be used to protect the side-chain primary amine moieties of lysine and ornithine. Allyloxycarbonyl groups can also be used to protect the guanido side-chain functional moiety of arginine. An allyl ether group can be used to protect the side-chain hydroxyl moieties of serine and threonine. An allyloxymethyl group, an allyl ether or an allyl thioether can be used to protect the sulfur-containing side-chain functional moieties of methionine and cysteine. An allyl ether can also be used to protect the phenolic moiety in tyrosine. In addition, the carboxylic acid or amide side-chain functional moieties of aspartic acid, glutamic acid, asparagine, and glutamine can be protected by an allyl ester or an allyl amide.

In each of the foregoing allyl side-chain protecting groups, the allyl moiety is of the general formula

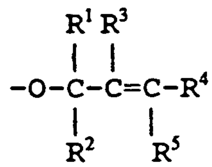

wherein $R^1$, $R^2$, $R^3$, $R^4$, and $R^5$ are independently selected from the group consisting of $C_1$ - $C_8$ linear or branched alkyl groups, $C_6$ - $C_{10}$ aryl groups, $C_1$ - $C_8$ linear or branched substituted alkyl groups, and $C_6$ - $C_{10}$ substituted aryl groups. In other words, the present alkyl or aryl groups can include as substituents, hydroxyl groups, ether linkages, ester linkages, thiols, thioether linkages, halogens, cyano groups, amines, nitro groups, sulfonyl groups, phosphonyl groups, and the like. An aryl group can also be substituted with an alkyl group, and conversely, an alkyl group can bear an aryl group as a substituent. In general, smaller R-groups are preferred, as the resulting allyl protecting group is non-bulky and does not produce steric hinderance in peptide bond coupling reactions during peptide synthesis. Allyl protecting groups are particularly preferred for solid-phase peptide synthesis, in that they confer favorable swelling (solvation) properties on the support-bound peptide under construction.

The allyl-protected amino acid derivatives of the present invention are produced by attaching an allyl side-chain protecting group to the side-chain of the amino acid and attaching an $^{\alpha}$N-protecting group to the $\alpha$ amino moiety of the amino acid. As noted previously, suitable $^{\alpha}$N-protecting groups are those which are orthogonal to the allyl group; carbonyl-type protecting groups are preferred. Methods and procedures for attaching suitable $^{\alpha}$N protecting groups to the $\alpha$ amino moiety of the amino acid are known to those skilled in the art and are described in M. Bodanszky, Peptide Chemistry: A Practical Textbook, Springer-Verlag (1988), RSPN No. 0-387-18984-X; available from Sigma Chemical Co., the teachings of which are incorporated by reference herein. For example, to attach an Fmoc protecting group, the amino acid or an allyl derivative thereof is contacted with 9-fluorenylmethyloxycarbonylsuccinate (Fmoc-OSu; the Fmoc derivative of *N*-hydroxysuccinimide) under basic conditions. The resulting compound, Fmoc-Xaa-OH or Fmoc-Xaa(OAl)-OH (wherein Xaa denotes the amino acid), is subjected to conditions sufficient to induce precipitation, or preferably, crystallization of the resulting $^{\alpha}$N-protected amino acid derivative. These procedures are more fully described in Example 1.

In one embodiment of the present method, allyl alcohol is used as the source of the allyl group, and sulfuric acid is used as a catalyst. The solid amino acid (e.g., in powder or crystalline form) is added to a solution of $H_2SO_4$ in allyl alcohol; this mixture is allowed to react to substantial completion. Progress of this reaction can easily be monitored by thin layer chromatography (TLC). A solvent system composed of ethyl acetate, pyridine, water and acetic acid has been found to provide good separation of the allyl-derivatized amino acid and the starting material, when run on aluminum-backed silica plates. When the reaction is observed to be substantially completed, the allyl-derivatized amino acid is subjected to conditions sufficient to result in precipitation or, preferably, crystallization of the derivative. In this manner, for example, the side-chain allyl esters of aspartic acid and glutamic acid can be produced; these procedures are the subject of Example 1(A) and 1(B), respectively. The side-chain allyl ethers of serine, threonine, and the phenolic ether of tyrosine, can also be produced, as can the side-chain thioether of cysteine and the side-chain allyloxymethyl derivative of methionine. As described in the Example, $^{\alpha}$N-protection can precede or follow allyl-protection: the preferred order of derivatization will depend on the particular amino acid derivative being prepared.

In another embodiment, the copper complex of the amino acid is prepared initially, then treated with allyloxycarbonyl chloride. The chloride salt of the amino acid is contacted with $CuSO_4$ under basic conditions, then treated with allylchloroformate in the presence of bicarbonate. The resulting crystals are treated with hydrogen sulfide to liberate copper, and the allyl-derivatized amino acid is subjected to conditions sufficient to result in precipitation or, preferably, crystallization of the derivative. In this manner, for example, the side-chain allyloxycarbonyl derivatives of lysine and ornithine (i.e., allyl amide linkages to the side-chain amino group) can be produced; these procedures are the subject of Example 1(C) and 1(D), respectively. Allyloxycarbonyl derivatives of arginine and histidine can be produced in a similar manner. It must be noted, however, that a peptidyl arginine residue can be enzymatically prepared from an allyl-protected ornithine derivative. Allyl amide-derivatives of asparagine and glutamine can be prepared as well. As with the first embodiment of the present method, $^{\alpha}$N-protection can either precede or follow allyl-protection.

The utility of the present allyl-protected amino acid derivatives in solid-phase peptide synthesis was demonstrated initially by preparing tetrameric peptides incorporating the instant allyl derivatives. Solid phase peptide synthesis typically begins with covalent attachment of the carboxyl end of a first, αN-protected amino acid derivative to a solid support. Solid supports which are useful for peptide synthesis include for example, membranes, porous glass, silica, polystyrenes, polydimethylacrylamides, cotton or paper. Solid supports having spacer arms attached thereto, referred to as "graft copolymers" are preferred in that such solid supports exert minimal steric effects (matrix effects) on peptide bond condensation reactions. Graft copolymers are described in J.K. Inman et al. (1977) Solid Phase Methods in Protein Sequence Analysis, INSERM Symp. No. 5, A. Previero and M.-A. Coletti-Previero, eds., Elsevier-North Holland, New York, p. 81-94; and in E. Bayer et al. (1983) Peptides: Structure and Function, Proc. 8th Am. Pept. Symp., V.J. Hruby and D.H. Rich, eds., Pierce Chemical Co., p. 87-90. The polyethylene glycol-polystyrene graft copolymers described in U.S.S.N. 07/576,634, filed August 31, 1990, are particularly preferred for use with the present allyl-protected amino acid derivatives.

The carboxyl group of the amino acid derivative is covalently linked to a handle moiety which is attached directly or through a spacer arm to the solid support. A "handle" is definded as a bifunctional spacer which serves to attach the initial amino acid residue in the sequence to be synthesized to the solid support. One end of the handle incorporates a smoothly cleavable protecting group, and the other couples to the solid support, typically via amine functional groups thereon. Handles which can be used for solid phase peptide synthesis include, for example acid-labile *p*-alkoxybenzyl (PAB) handles, photolabile *o*-nitrobenzyl ester handles, and handles such as those described in F. Albericio et al. (1990) J. Org. Chem. **55**:3730-3743, U.S.S.N. 07/576, 232, filed August 31, 1990, and U.S.S.N. 07/576,233, also filed on that date. Acid-labile handles are used in the procedures described in Examples 2 - 4. The appropriate handles are coupled quantitatively in a single step onto the amino-functionalized supports to provide a general starting point of well-defined structures for peptide chain construction. The C-terminal $^{\alpha}$N-protected amino acid derivative is then coupled quantitatively to the handle. The support-bound amino acid derivative is $^{\alpha}$N-deprotected, the support is washed and, if necessary, neutralized. The peptide chain elongation synthesis cycle then generally proceeds.

The synthesis cycle consists generally of reaction of a $^{\alpha}$C-activated, $^{\alpha}$N-protected amino acid derivative with the support-bound, $^{\alpha}$N-deprotected terminal amino acid residue in the growing chain, followed by washing, neutralization, and $^{\alpha}$N-deprotection. The cycle is repeated with the appropriate amino acid derivatives to form the sequence of interest. Activating agents and reaction conditions for peptide bond condensation are described generally in G. Barany (1987) Int. J. Pept. Prot. Res. **30:**705-739, at 722-724. Benzotriazolyl *N*-oxytrisdimethyamino-phosphonium hexafluorophosphate in conjunction with 1-hydroxybenzotriazole (BOP/HOBT) are particularly preferred activation agents for coupling reactions, and are described in Example 2. The use of the BOP/HOBT system is more particularly described in D. Hudson, (1988) J. Org. Chem. **53**:617-624, the teachings of which are incorporated herein by reference.

The results of Example 2 demonstrate that each of the allyl side-chain protected amino acid derivatives prepared in Example 1 can be incorporated into a peptide constructed by solid phase synthesis. Allyl side-chain protection is particularly advantageous in solid phase peptide synthesis, in that the side-chain protecting groups are non-bulky and thereby do not present steric barriers to the condensation of peptide bonds adjacent to the protected amino acid residue in the chain, or to the condensation of allyl-protected amino acid derivatives with the support-bound peptide under construction. The allyl group, unlike the hydrophobic *t*Boc or the bulky, aromatic trityl side-chain protecting groups, promotes swelling of resinous supports, and solvation of the support-bound peptide. As a result, peptide peptide bond condensation during chain elongation proceeds readily.

The resulting support-bound peptide can be allyl deprotected through the use of a suitable noble metal organometallic catalyst, such as *tetrakis*-triphenylphosphine palladium(0), in the presence of a nucleophilic acceptor, such as morpholine. The use of organopalladium catalysts for reactions involving allyl groups is described in B.M. Trost (1977) Tetrahedron **33:**2615-2649. The deprotection reaction proceeds under mild, neutral conditions which are nondamaging to the support-bound peptide. Thus, allyl deprotection does not affect the stability of other types of protecting groups, such as Fmoc and *t*Boc. Conversely, the allyl group has been found to remain stable to the conditions generally encountered in deprotection of αN-protecting groups. Furthermore, allyl deprotection, unlike the removal of side-chain protecting groups such as *tert*-butyl, benzyl or trityl groups, does not result in the formation of reactive carbonium ion elimination products. Thus, allyl deprotection does not present the risk of undesired side-reactions, such as the alkylation of tyrosine, tryptophan, cysteine or methionine residues, encountered with the use of other side-chain protecting groups.

It was found that allyl deprotection proceeded slowly on simple polystyrene resins; this apparant

limitation can be circumvented by repeated or prolonged exposure of the support-bound peptide to the deprotection catalyst, as product integrity is not impaired thereby. However, polar or graft copolymers, such as the polyethylene glycol-polystyrene graft copolymer described previously and employed in Examples 2 - 4, do not exert this effect: allyl deprotection proceeds rapidly and quantitatively.

The utility of the present allyl side-chain protected amino acid derivatives in solid-phase peptide synthesis was further illustrated by the successful construction and quantitative deprotection of the nonapeptide, prothrombin 1-9. These results are more fully described in Example 3.

The allyl-protected amino acid derivatives of the present invention are additionally useful in solid-phase peptide synthesis in that they provide means to construct cyclic or branched peptide structures. For these purposes, allyl side-chain protection according to the present invention can be combined with other side-chain protection strategies to produce peptides wherein selected side-chain moieties can be deprotected and further derivatized while all other protection remains unaffected. In this manner, for example, an entirely different peptide sequence can be constructed from the $^{\epsilon}$N-moiety of lysine. Branched peptides constructed in this manner can be particularly useful as peptide immunogens for the production of antibodies, as two or more linear peptide epitopes can be combined, facilitating antigen presentation, or a nonlinear epitope (e.g., one produced by protein folding) can be constructed.

Cyclic peptides are also useful for such purposes, and can be constructed using a strategy of allyl side-chain protection at the sites at which, for example, an intra-peptide side chain-to-side chain amide bond is to be introduced. A.M. Felix et al., (1988) Int. J. Pept. Prot. Res. **31**:231-238 describe a method for producing such an amide-bonded cyclic peptide through the use of BOP as a cyclizing agent. The use of allyl side-chain protection in conjunction with cyclization is more fully described in Example 4, wherein the results of synthesis of a cyclic human growth hormone releasing factor peptide are presented. Circularization or cyclization of peptide drugs is advantageous in that stable configurations of the peptide can be preserved, leading to greater functional activity. In addition, cyclic peptides are more resistant to degradation, resulting in extended *in vivo* half-life of the therapeutic.

The invention will now be further illustrated by the following examples, which are not to be viewed as limiting in any way.

EXAMPLE 1: SYNTHESIS OF ALLYL-SIDE CHAIN PROTECTED $^{\alpha}$N-FMOC AMINO ACID DERIVATIVES

A. SYNTHESIS OF FMOC-ASP(OAL)-OH.

The synthesis of each Fmoc-amino acid involved two steps. In the case of aspartate, the allyl side chain protecting group was attached initially, forming Asp-allyl ester. This reaction was carried out in allylic alcohol, using $H_2SO_4$ as a catalyst:

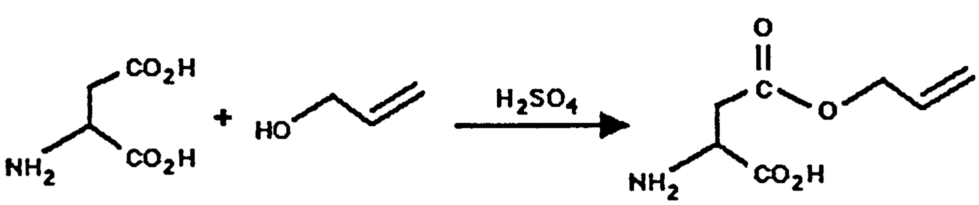


Eq(1)

Asp(OAl)-OH: One liter of anhydrous ether was cooled on ice under argon with stirring in a 3.0 L flask. Concentrated $H_2SO_4$ [60 mL] was slowly added to the flask, followed by allyl alcohol [300 mL; 4.4 moles] (source, Aldrich Chemical Co.). The ether was removed by rotary evaporation. Aspartic acid [60 g; 0.45 moles] was added to the flask, and the cloudy mixture was stirred overnight. The next day, the solid was found to have dissolved.

Thin layer chromatography (TLC, using a solvent system of ethyl acetate: pyridine: water: acetic acid in the proportions 35:20:11:6, vol:vol) of the mixture revealed good progress of the reaction, with only a small amount of underivatized aspartic acid remaining.

The stirring bar was removed from the flask, and the excess allyl alcohol was removed by rotary evaporation. The resulting light brown syrup was dissolved in 1.0 L of HPLC-grade water, and KOH pellets were added until a pH of 5.0 was attained. The product, a solid precipitate, was removed by filtration, and the filtrate was concentrated to about 500 mL. An equal volume (500 mL) of methanol was added, and the resulting slurry was filtered. The filtrate was concentrated to 120 g of a tar.

For preparative purposes, the ester product was not isolated. Instead, the crude product was treated with 9-fluorenylmethyloxycarbonyl-succinate (Fmoc-OSu; the Fmoc derivative of *N*-hydroxysuccinimide) at pH 9. Acidification and extraction yielded Fmoc-Asp(OAl)-OH, shown below.

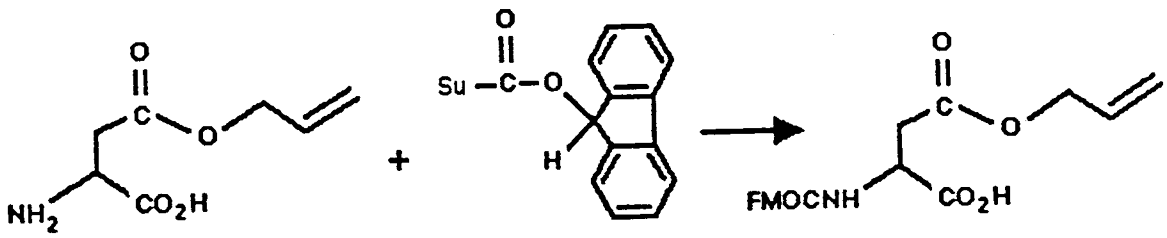

(FMOC-OSu)

Eq(2)

Fmoc-Asp(OAll)-OH: The tar obtained in step 1, above, was dissolved in 500 mL of dilute aqueous KOH and the final pH was adjusted to 10 with solid KOH. Fmoc-OSu [68 g; 0.201 moles] was added in small portions over 1 hour, while the pH was maintained between 8 and 10 with solid KOH. The reaction was stirred for an additional 30 minutes after the final addition of Fmoc-OSu, and was poured into 200 mL of ice containing 50 mL of concentrated HCl. The resulting white suspension was extracted twice with ethyl acetate, and the combined organic phases were washed once with 1.0 N HCl, dried over $Na_2SO_4$, and concentrated to a tar. This residue was applied to a silica column (10x30 cm), and eluted with a step gradient (500 mL increments) of 1% to 10% methanol in methylene chloride.

The purest fractions (having a retention factor (rf) of 0.15 upon TLC in a solvent system of 10% methanol in methylene chloride) were evaporated, and the residue was dissolved in ethyl acetate and cooled to 4°C. Nine and one-half grams (9.5 g, representing a 7.3% yield from the starting material, aspartic acid) of white crystals, mp 105 - 108°C, were collected.

Analysis of the product by nuclear magnetic resonance (NMR), Fourier-transforming infra red spectroscopy (FT-IR), and compositional analysis indicated that the first reaction (addition of the allyl protection group) showed remarkable selectivity, with only small amounts of the $^{\alpha}$C-regioisomer or the disubstituted product observed.

$^1$H NMR (200 MHZ , $CDCl_3$), ppm:
8.0 - 7.0, (multiplet, m) 8 H; 6.5 - 6.0, (m) 1 H; 5.7 - 5.5, (m) 1 H; 5.3 - 4.9, (m) 2 H; 4.6 - 3.9, (m) 5 H ; 3.0 - 2.6, (m) 1 H .
$^{13}$C NMR (50 mHz, $CDCl_3$) ppm:
145.7, 130.8, 126.8, 126.1, 124, 118.8, 117.4, 66.5, 64.9, 50.9, 46.0, 36.0.
FT-IR, $cm^{-1}$:
3395, 3171, 1757, 1738, 1716, 1514, 1219, 1211.
Analysis calculated for $C_{22}H_{21}NO_6$:
C, 66.83. H, 5.35. N, 3.54.
Analysis found:
C, 66.97. H, 5.66. N, 3.38.

B. SYNTHESIS OF FMOC-GLU(OAL)-OH.

The reaction sequence described above for Asp did not prove efficacious for glutamate; prior attachment of the Fmoc group, yielding Fmoc-Glu-OH, proved to be a more successful strategy.

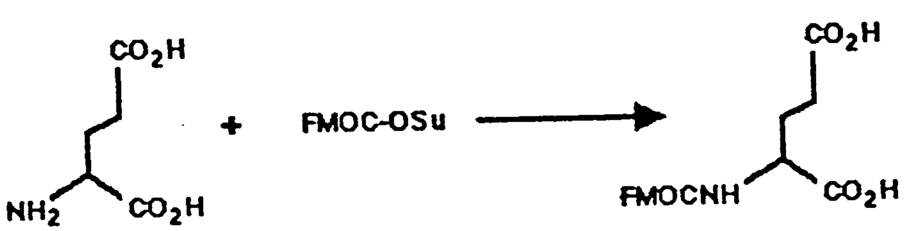


Eq(3)

Fmoc-Glu-OH. A solution containing glutamic acid [20 g; 0.136 moles] in 300 mL of tetrahydrofuran (THF)

and 300 mL of water was adjusted to pH 9 with KOH pellets and 1 M KOH solution. Fmoc-OSu [45 g; 0.134 moles] was added in 5 portions with stirring. A small amount of 1 M KOH solution was added 5 min after each addition, to restore a pH of 9 in the reaction mixture. When all of the Fmoc-OSu had been added, the suspension, which had been cloudy, became clear. It was poured into 100 mL of concentrated HCl in 500 mL of ice. The resulting white suspension was extracted with 500 mL ethyl acetate and dried over $Na_2SO_4$. This solution was filtered and evaporated to 200 mL, whereupon hexane was added until the mixture became cloudy. This mixture was chilled, producing crystals which were collected and dried to give 34.5 g (70% yield from glutamic acid) of white crystals, mp 185 - 188 ° C.

The product was analyzed by NMR, IR, mass spectroscopy, and compositional analysis; results are shown below.

$^{13}C$ NMR (50 MHz, $CDCl_3$), ppm:
26.2, 30.2, 46.7, 53.0, 66.0, 120.1, 125.2, 127.1, 127.7, 140.9, 143.9, 156.2, 173.8, 173.4.
FT-IR, $cm^{-1}$:
3306, 1711, 1695, 1547, 1446, 1269, 1059.
M/e (relative intensity):
370(44), 307(46), 289(25), 246(10), 219(20), 192(14), 179(100), 165(24).
Analysis calculated for $C_{20}H_{19}NO_6$:
C, 64.98. H, 5.17. N, 3.85.
Analysis found:
C, 64.61. H, 4.98. N, 3.84.

Treatment of the product of step one with $H_2SO_4$ in allyl alcohol resulted in the formation of the desired Fmoc-Glu(OAll)-OH, shown below. Some disubstituted ester was also formed, and was separated from the desired product by acid-base extraction.

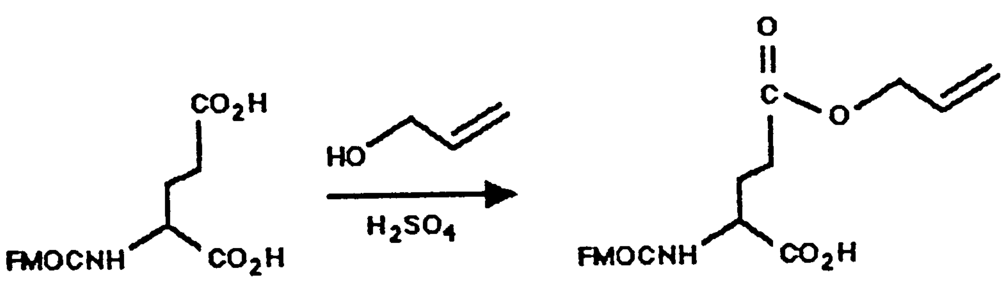


Eq (4)

Fmoc-Glu(Al)-OH. Fmoc-Glu-OH [10 g; 27 millimoles] from step 1 was stirred with 100 mL of allyl alcohol, and 7 mL of concentrated $H_2SO_4$ was added. The mixture was stirred for 1 hr, during which time most of the solid dissolved. Ether [500 mL] was added, and the mixture was washed twice with 500 mL of water. The ether layer was washed with 200 mL of 0.1 M NaOH solution. This layer was drained into 10 mL of concentrated HCl in 200 mL of ice, which gave a white suspension. This material was extracted with 200 mL of ethyl acetate, dried over $Na_2SO_4$ and reduced in volume to about 100 mL. Hexane was added until the solution became cloudy, and overnight chilling provided 2 g (18% yield from the product of step one) of white crystals, mp 54 - 57 ° C.

The product was analyzed by NMR, IR, mass spectroscopy, and compositional analysis; results are shown below.

$^{1}H$ NMR (200 MHz, $CDCl_3$) ppm:
8.7 - 8.3, (m) 2H; 7.8 - 7.2, (m) 8 H; 6.0 - 5.2, (m) 3 H; 4.6 - 4.2, (m) 6 H; 2.6 - 1.8, (m) 4 H.
$^{13}C$ NMR (50 MHz, $CDCl_3$) ppm:
27.2, 30.0, 47.2, 53.4, 65.6, 67.4, 118.6, 119.9, 125.0, 127.1, 127.7, 131.8, 141.2, 143.5, 156.2, 172.7, 175.7.
FT-IR, $cm^{-1}$:
3323, 1734, 1691, 1540, 1260, 1175, 1042.
M/e (relative intensity):
410(66), 355(14), 188(15), 170(100), 165(16), 130(10).
Analysis calculated for $C_{23}H_{23}NO_6 \cdot 1/2\ H_2O$:
C, 66.02. H, 5.54. N, 3.35
Analysis found:
C, 65.85. H, 5.66. N, 3.04.

C. SYNTHESIS OF FMOC-LYS(ALOC)-OH.

For the protection of the $^{\epsilon}$N-primary amine moiety of lysine, the copper complex of the amino acid was prepared initially, then treated with allyloxycarbonyl chloride. The product, a blue precipitate, was collected, and treated with $H_2S$ to remove the copper. Upon concentration and cooling, white crystals of lysine-4-allyloxycarbonamide, Lys(AlOC)-OH, were collected:

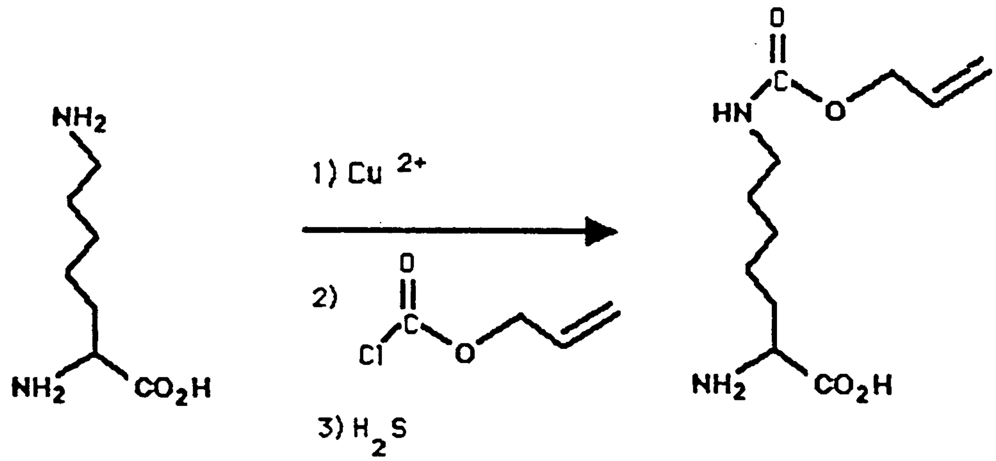


Eq(5)

Lys(AlOC)-OH. Lysine hydrochloride [91.5 g; 0.5 mole] was dissolved in a solution of 12 g of NaOH in 1 L water. To this was added $CuSO_4$ [62.4 g; 0.25 moles], and the solution was stirred until all of the $CuSO_4$ had dissolved, producing a dark blue solution. Solid $NaHCO_3$ [150 g] was added, followed by a solution of allyl chloroformate [66 g; 0.55 moles) in 100 mL dioxane. The solution bubbled, and was stirred overnight. The mixture was filtered, and the precipitate was washed with dioxane, leaving a sky blue solid. Another 20 g of allyl chloroformate was added to the mother liquor, and more blue solid was obtained after 4 hours. The combined solids were placed in a 4 L Erlenmeyer flask with a large stirbar, and 2 L water was added. The mixture was heated on a hot plate with stirring, and $H_2S$ was bubbled through (obtained by slow addition of concentrated HCl to FeS). The mixture turned black, and after 2 hours, no blue color could be seen. The mixture was filtered carefully through a paper filter in a large Hirsch filter, and the yellow solution was allowed to cool and outgas for 24 hours. It was reduced on the rotary evaporator until crystals appeared, and then cooled to 4 °C overnight. White crystals were obtained. The mother liquor was concentrated twice more, and two additional crops of crystals were obtained. The crops of crystals appeared equivalent to each other by TLC analysis (using the solvent system ethyl acetate: pyridine: water: acetic acid of 35:20:11:6, vol/vol). The combined yield was 38 g (33% from the starting material, Lys-HCl). A small amount of this material was recrystallized from water, mp 182 - 190 °C.

The product was analyzed by NMR and compositional analysis; results are shown below.

$^{1}$H NMR (200 MHz, $D_2O$)ppm:

6.0 - 5.7 (m) 1 H; 5.3 - 5.0 (m) 2 H; 4.5 - 4.4 (doublet, d) J = 6 Hz, 2 H; 3.7 - 3.5 (triplet, t) J = 8 Hz, 1 H; 3.1 - 2.9 (t) J = 7 Hz, 2 H; 1.9 - 1.7 (m) 2 H; 1.5 - 2.5 (m) 4 H.

Analysis calculated for $C_{10}H_{16}N_2O_4$:

C, 52.6. H, 7.87. N, 12.16.

Analysis found:

C, 51.72. H, 8.11. N, 12.35.

The crystalline product, Lys(AlOC)-OH, was $^{\alpha}$N-protected by reaction with Fmoc-OSu at pH 9, generating Fmoc-Lys(AlOC)-OH. The desired product was recovered in crystalline form after extraction and workup by standard procedures:

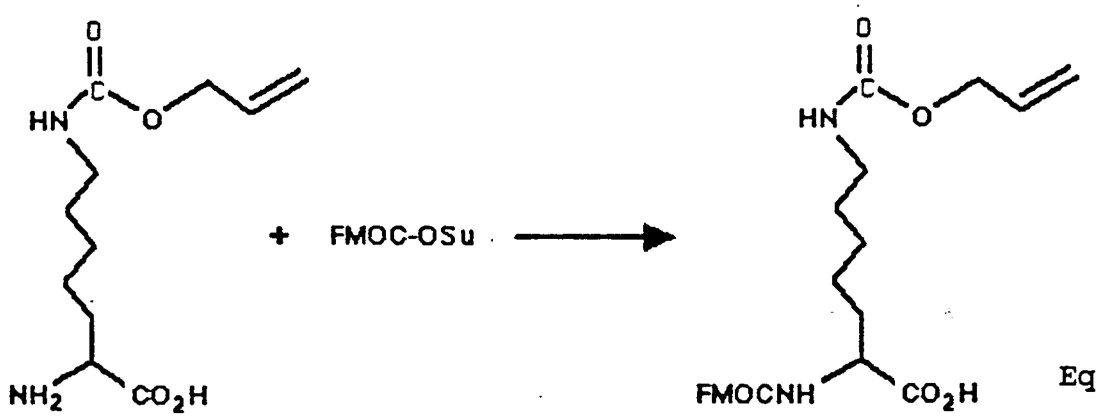


Eq(6)

Fmoc-Lys(AlOC)-OH. The crystalline product of step one above [28 g; 0.121 moles] was dissolved in a mixture of 500 mL of water and 500 mL of dioxane, and KOH pellets were added until a pH of 10 was attained. To this mixture, Fmoc-OSu [36.5 g; 0.108 moles] was added in small portions, and additional KOH was added from a 1 M solution, to maintain a pH between 9 and 10. Two hours after the last of the Fmoc-OSu was added, the mixture was poured into 200 mL of ice and 50 mL of concentrated HCl. The white mixture was extracted twice with 500 mL of ethyl acetate, and the combined organic phases were washed with 500 mL of 1 N HCl and dried over $Na_2SO_4$. The solution was reduced to a tar and immediately chromatographed through a silica column, from which it was eluted with ethyl acetate. Seven grams of pure material, having an rf of 0.1 upon TLC using a silica plate and a solvent system of 10% methanol in methylene chloride, was obtained, along with a fraction which contained unreacted Fmoc-OSu. The crude product was crystallized from hexane-saturated ethyl acetate (hereinafter ethyl acetate/hexane) to give 11.8 g of pure material. The total yield was 18.8 g (38%, based upon Fmoc-OSu as the starting material). A small amount of this was recrystallized from ethyl acetate/hexane mp 80 - 85 ° C.

The product was analyzed by NMR, IR, mass spectroscopy, and compositional analysis; results are shown below.

$^{13}C$ NMR (50 MHz, $CDCl_3$) ppm:

175.7, 143.7, 143.5, 141.1, 132.7, 127.6, 126.9, 125.0, 119.9, 117.6, 66.9, 65.5, 53.5, 46.9, 40.3, 31.6, 29.1, 22.1.

M/e (relative intensity):

107(37), 121(20), 136(68), 154(64), 165(27), 178(100), 191(14), 231(15), 295(13), 453(21), 491(19, K+ salt).

FT-IR, $cm^{-1}$:

3335, 2943, 1743, 1695, 1649, 1531, 1263.

Analysis calculated for $C_{25}H_{28}N_2O_6$:

C, 66.36. H, 6.24. N, 6.19.

Analysis found:

C, 66.18. H, 6.23. N, 6.06.

C. SYNTHESIS OF FMOC-ORN(ALOC)-OH.

This amino acid derivative was prepared in the manner described above for lysine.

Orn(AlOC)-OH. Ornithine hydrochloride [60 g; 0.375 moles] was dissolved in 800 mL of water and 9 g of NaOH was added. $CuSO_4$ [47 g; 0.1875 moles] was added to this solution, whereupon the mixture became deep blue in color; it was stirred until all solids had dissolved. Next, 100 g of $NaHCO_3$ was added, followed by allyl chloroformate [56.8 g; 0.4125 moles) in 300 mL of dioxane. The mixture bubbled and was stirred overnight. A sky blue solid was collected on a paper filter, and suspended in 2 L of hot water. $H_2S$ was bubbled through for 3 hours, and the black suspension was filtered and allowed to stand for 24 hours. The resulting yellow solution was concentrated to 500 mL and chilled overnight. White crystals were obtained and collected and washed with cold water. The mother liquor was further concentrated, yielding a second crop of crystals. Both batches appeared identical by TLC analysis (solvent system of ethyl acetate: pyridine: water: acetic acid, 35:20:11:6, vol/vol). The yield was 41.9 g (51.5% from Orn-HCl), mp 260 ° C (decomposed).

The product was analyzed by NMR and compositional analysis; results are shown below.

$^1H$ NMR (200 MHz, $D_2O$) ppm:

6.0 - 5.7 (m) 1 H; 5.3 - 5.0 (m) 2 H; 4.5 - 4.4, (d) J = 5 Hz, 2 H; 3.7 - 3.5 (t) J = 7 Hz, 1 H; 3.2 - 3.0 (t) J = 6 Hz, 2 H; 1.9 - 1.6 (m) 2 H; 1.6 - 1.3 (m) 2 H.
Analysis calculated for $C_8H_{16}N_2O_4$:
C, 49.99. H, 7.45. N, 12.95.
Analysis found:
C, 50.09. H, 7.56. N, 13.14.
Fmoc-Orn(AlOC)-OH. The product of step one [11 g; 0.051 moles] was dissolved in a mixture of 200 mL of water and 200 mL of dioxane, and KOH pellets were added until a pH of 10 was attained. Fmoc-OSu [15 g; 0.045 moles] was added in small portions, and additional KOH was added as a 1 M solution, to maintain a pH between 9 and 10. Two hours after the last of the Fmoc-OSu was added, the mixture was poured into 200 mL of ice and 50 mL of concentrated HCl. The white mixture was extracted twice with 300 mL of ethyl acetate, and the combined organic phases were washed with 200 mL of 1 N HCl and dried over $Na_2SO_4$. The solution was reduced to a tar, and 100 mL of ethyl acetate was added, along with sufficient hexane to produce cloudiness in the solution. The cloudy solution was cooled to 4°C overnight, and white crystals were collected and washed with cold ethyl acetate. The total yield was 12.7 g (57% based upon Fmoc-OSu as the starting material). A small amount of this product was recrystallized from ethyl acetate/hexane, mp 82 - 85°C.

The product was analyzed by NMR, IR, mass spectroscopy, and compositional analysis; results are shown below.
$^1H$ NMR (200 MHz, $CDCl_3$) ppm:
7.8 - 7.1 (m) 8 H; 5.8 - 5.6 (m) 1 H; 5.4 - 5.2 (m) 2 H; 4.6 - 4.2 (m) 5 H; 3.4 - 3.2 (m) 2 H; 2.0 - 1.3 (m) 4H.
$^{13}C$ NMR (50 MHz, $CDCl_3$) ppm:
156.7, 143.7, 143.5, 141.1, 132.6, 127.6, 126.9, 125.0, 119.8, 117.7, 67.0, 65.6, 53.8, 47.0, 40.3, 29.3, 25.8.
FT-IR, $cm^{-1}$:
3331, 3067, 1695, 1539, 1255.
Analysis calculated for $C_{24}H_{26}N_2O_6$:
C, 65.74. H, 5.98. N, 6.39.
Analysis found:
C, 65.73. H, 5.70. N, 6.12.

Summary: Properties of Amino Acid Derivatives.

Small amounts of each of the above-described amino acid derivatives were recrystallized several times from ethyl acetate/hexane, and subjected to melting point and compositional analysis. The results of these determinations are presented in the text above and summarized in Table 2, below. In addition, the derivatives were analyzed by high pressure liquid chromatography (HPLC), using a Waters Microbond™-pack $C_{18}$ analytical column (Waters Chromatography Division, Millipore, Bedford MA) with the following buffer system: A = acetonitrile, B = 0.1% TFA in water, linear gradient of 5 - 100% B over 20 minutes), and thin-layer chromatography (TLC), using aluminum-backed silica plates (source, EM reagents, catalog no. 5534) with solvent systems as indicated in the Table.

Table 2

| Properties of Amino Acid Derivatives | | | | |
|---|---|---|---|---|
| Compound | mp °C | MWt | retention, HPLC (min.) | rf, TLC |
| Fmoc-Asp(OAl)-OH | 105-108 | 395.4 | 16.0 | 0.47[a] |
| Fmoc-Glu(OAl)-OH | 66-72 | 409.4 | 16.5 | 0.49[a] |
| Fmoc-Lys(AlOC)-OH | 80-85 | 452.5 | 15.7 | 0.65[b] |
| Fmoc-Orn(AlOC)-OH | 82-85 | 438.4 | 14.0 | 0.63[b] |

[a] solvent system of chloroform: methanol: acetic acid, 77.5:15:7.5
[b] solvent system of chloroform: methanol: acetic acid, 90:8:2

## EXAMPLE 2: SYNTHESIS AND DEPROTECTION ANALYSIS OF TEST PEPTIDES

Test peptides incorporating the allyl-side chain protected, $^{\alpha}N$-Fmoc-amino acid derivatives described in

Example 1 were synthesized on a Milligen/Biosearch Peptide Synthesizer, model 9600 (Milligen/Biosearch Division, Millipore, Novato CA). The activation reagents used for coupling were benzotriazolyl *N*-oxytrisdimethyamino-phosphonium hexafluorophosphate in conjunction with 1-hydroxybenzotriazole (BOP/HOBT). D. Hudson, (1988) J. Org. Chem. **53**:617-624, the teachings of which are incorporated herein by reference. See also G. Barany et al. (1987) Int. J. Pept. Prot. Res. **30:**705-739 at 722-723, and A.M. Felix et al., (1988) Int. J. Pept. Prot. Res. **31**:231-238, at 232. Diisopropylcarbodiimide (DIPCDI) activation was also used (See Barany et al. at 722).

For each test peptide, 1 g of Fmoc-PAL-PEG-MBHA resin, [5'-(4''-(9-fluorenylmethyloxycarbonyl) aminoethyl-3,5-dimethoxyphenoxy) valeric acid]-polyethylene glycol-*p*-methylbenzyhydrylamine polystyrene resin (described in copending U.S.S.N. 07/576,634, filed August 31, 1990, the teachings of which are incorporated by reference herein) was used at a loading ratio of 0.13 - 0.17 millimoles peptide per gram of resin. The allyl-protected amino acid derivatives were incorporated into the test sequence isoleucine-alanine-(allyl-derivatized amino acid residue)-glycine [Ile-Ala-Xaa(Al)-Gly], using 4 hour coupling times to ensure complete incorporation. Each of the four test peptides were prepared as C-terminal amides, and the test sequences were confirmed by amino acid analysis (AAA).

Samples (100 - 500 mg) of the resin-bound peptides, still bearing the N-terminal Fmoc protecting group, were placed in 5 mL plastic vials with tight-fitting caps. Two hundred fifty milligrams of triphenylphosphine were added to each vial, followed by 0.2 mL of morpholine (which had been refluxed over and distilled from $CaH_2$). Subsequently, 3 mL of dry (freshly distilled from Na/benzophenone) THF was added, followed by 50 mg of the catalyst, *tetrakis*-triphenylphosphine palladium(0) (Alfa Chemical Co.). B.M. Trost (1977) Tetrahedron **33**:2615-2649. See also, H. Kunz, (1987) Angew. Chem. Int. Ed. Engl. **26**:294-308, at 304-306, and H. Kunz and H. Waldmann, (1984) Angew. Chem. Int. Ed. Engl. **23**:71-72. The mixture was shaken overnight, and the resin was washed several times with THF before the N-terminal Fmoc group was removed with 20% piperadine in dimethylformamide (DMF). Subsequently, the tetrapeptides were cleaved from the supporting resin with TFA, and recovered from solution.

The extent (smoothness) of deprotection of the allyl-derivatized amino acid residues was analyzed by thin layer chromatography (solvent system used, ethyl acetate: pyridine: water: acetic acid, 35:20:11:6, vol/vol) on aluminum-backed silica plates. Control deprotected peptide tetramers were prepared according to standard methods, using *t*Boc-side chain derivatized amino acids, and were used to establish rf values for the deprotected test peptides. Rf values for the protected and deprotected tetrapeptides are shown in Table 3.

Table 3

| Rf values of test tetrapeptides | | | |
|---|---|---|---|
| Protected | rf | Deprotected | rf |
| Ile-Ala-Asp(OAl)-Gly | 0.78 | Ile-Ala-Asp-Gly | 0.28 |
| Ile-Ala-Glu(OAl)-Gly | 0.62 | Ile-Ala-Glu-Gly | 0.47 |
| Ile-Ala-Lys(AlOC)-Gly | 0.78 | Ile-Ala-Lys-Gly | 0.07 |
| Ile-Ala-Orn(AlOC)-Gly | 0.71 | Ile-Ala-Orn-Gly | 0.07 |

Following palladium-catalysed allyl removal for each tetrapeptide, no visible trace of the protected peptide remained upon TLC analysis. Fast-atom bombardment (FAB) mass spectrometry of the palladium-treated tetrapeptides confirmed that the allyl protecting group was no longer associated with the peptide, indicating better than 95% efficiency in removal.

In order to verify that the allyl protecting groups remained in place during repeated cycles of Fmoc deprotection while the peptides were under construction, samples of the allyl-derivatized amino acids were subjected to neat piperidine treatment for 24 hours, and subsequently analyzed by TLC. In each case, the major spots observed were due to the intact allyl derivatives. Integrity of the allyl-amino acid derivatives during peptide elongation was confirmed by the absence of branching observed upon FAB-MS and AAA analysis.

EXAMPLE 3: SYNTHESIS OF PROTHROMBIN 1-9 USING ALLYL-PROTECTED AMINO ACID DERIVATIVES

The nonapeptide prothrombin 1-9, having the sequence Ala-Asn-Lys-Gly-Leu-Phe-Glu-Glu-Val-$NH_2$, was

synthesized using the Fmoc-Glu(Oall)-OH and Fmoc-Lys(Alloc)-OH amino acid derivatives described above in Example 1. The synthesis strategy detailed in Example 2 was employed: BOP/HOBt mediated peptide bond condensation, followed by allyl deprotection, N-terminal Fmoc removal, and TFA cleavage from the resin. The resulting nonapeptide was analyzed by FAB-MS and reverse-phase HPLC. The mass spectrograph revealed that all three allyl groups employed in constructing the sequence had been completely removed, while the HPLC elution profile indicated that a peptide in excess of 90% purity had been obtained.

EXAMPLE 4: SYNTHESIS OF THE CYCLIC PEPTIDE HUMAN GROWTH HORMONE RELEASING FACTOR 1-29[ASP 27 - LYS 18] USING ALLYL-PROTECTED AMINO ACID DERIATIVES

The complete sequence of human growth hormone releasing factor 1-29, Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Asp-Ile-Met-Ser-Arg-$NH_2$, was synthesized using the *tert*-butyl protected Fmoc derivatives of all amino acid side chain functional moieties except Asp 27 and Lys 18, for which the allyl derivatives described in Example 1 were employed. A synthesis strategy similar to that described in Example 2 was employed, and following allyl deprotection, the resin-bound peptide was subjected to treatment with BOP reagent to induce [Asp 27:Lys 18] side chain-to-side chain coupling according to the method of A.M. Felix et al., (1988) Int. J. Pept. Prot. Res. **31**:231-238. Following TFA-mediated cleavage from the resin, the resulting cyclic peptide was analyzed by FAB-MS and Edman degradation (N-terminal sequence analysis) according to standard procedures. The sequence profile exhibited missing residues at positions 18 and 27, owing to the attached (27) or modified (18) nature of the phenylthiohydantoin (PTH) amino acids produced by the Edman peptide sequencing reactions. A sequence profile generated from a sample which had not been subjected to treatment with BOP reagent was found to include an Asp residue at position 27 and Lys at position 18. Mass spectrograph results indicated that the apparent gaps in the sequence of the circularized peptide were due to side chain-to-side chain coupling rather than incomplete allyl deprotection.

EQUIVALENTS

Those skilled in the art will recognize or be able to ascertain, using no more than routine experimentation, many equivalents to the specific embodiments of the invention described herein. These and all other such equivalents are intended to be encompassed by the following Claims:

## Claims

1. An amino acid derivative for peptide synthesis, in which the side chain functional moiety thereof is protected with an allyl side-chain protecting group.

2. The side-chain protected amino acid derivative of Claim 1 wherein the allyl side-chain protecting group is selected from the group consisting of allyloxycarbonyl, allyloxymethyl, allyl ether, allyl thioether, allyl ester, and allyl amide.

3. A compound of the general formula

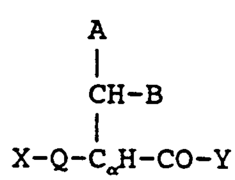

wherein the alpha carbon is either of the L or D configuration; Y is selected from the group consisting of a halogen, a hydroxyl group, and an active ester; X is a carbonyl-type protecting group; Q is an a amino group, in either di- or tri-substututed form; B is a hydrogen or a methyl group; and A is an amino acid side-chain protected by an allyl side-chain protecting group.

4. A compound of Claim 3 wherein the active ester is selected from the group consisting of pentafluorophenyl, nitrophenylpyrazol, benzotrialzolyloxy, and active intermediates generated by coupling agents, including O-acylisourea and symmetrical anhydrides.

**5.** A compound of Claim 3 wherein the carbonyl-type protecting group is selected from the group consisting of *tert*-butyloxycarbonyl, *tert*-amyloxycarbonyl, adamantanyloxycarbonyl, 9-fluorenylmethyloxycarbonyl, 2-(3,5-dimethoxyphenyl)-2-propyloxycarbonyl, dithiasuccinoyl, biphenylylisopropyloxycarbonyl, and phenylisopropyloxycarbonyl.

**6.** A compound of Claim 3 wherein the allyl-protected amino acid side-chain is represented by the general formula

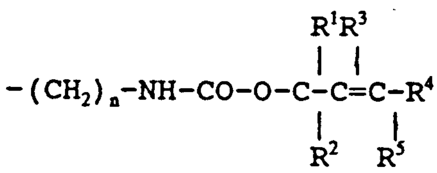

wherein n is from an integer 1 to 4; $R^1$, $R^2$, $R^3$, $R^4$, and $R^5$ are independently selected from the group consisting of hydrogen, $C_1$ - $C_8$ linear or branched alkyl groups, $C_6$ - $C_{10}$ aryl groups, $C_1$ - $C_8$ linear or branched substituted alkyl groups, and $C_6$ - $C_{10}$ substituted aryl groups.

**7.** An amino acid derivative of Claim 1, wherein the amino acid is selected from the group consisting of lysine and ornithine, and the side-chain protecting group is an allyloxycarbonyl group.

**8.** A compound of Claim 3 wherein the allyl-protected amino acid side-chain is represented by the general formula

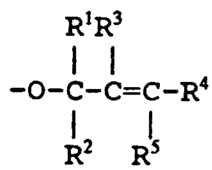

wherein $R^1$, $R^2$, $R^3$, $R^4$, and $R^5$ are independently selected from the group consisting of $C_1$ - $C_8$ linear or branched alkyl groups, $C_6$ - $C_{10}$ aryl groups, $C_1$ - $C_8$ linear or branched substituted alkyl groups, and $C_6$ - $C_{10}$ substituted aryl groups.

**9.** An amino acid derivative of Claim 1, wherein the amino acid is selected from the group consisting of serine and threonine, and the side-chain protecting group is an allyl ether.

**10.** A compound of Claim 3 wherein the allyl-protected amino acid side-chain is represented by the general formula

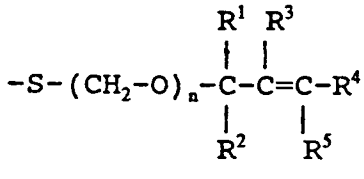

wherein n is 0 or 1; and $R^1$, $R^2$, $R^3$, $R^4$, and $R^5$ are independently selected from the group consisting of hydrogen, $C_1$ - $C_8$ linear or branched alkyl groups, $C_6$ - $C_{10}$ aryl groups, $C_1$ - $C_8$ linear or branched substituted alkyl groups, and $C_6$ - $C_{10}$ substituted aryl groups.

**11.** An amino acid derivative of Claim 1, wherein the amino acid is selected from the group consisting of methionine and cysteine, and the side-chain protecting group is selected from the group consisting of allyloxymethyl, allyl ether and allyl thioether.

**12.** A compound of Claim 3 wherein the allyl-protected amino acid side-chain is represented by the general formula

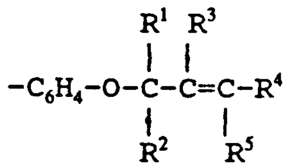

wherein $R^1$, $R^2$, $R^3$, $R^4$, and $R^5$ are independently selected from the group consisting of hydrogen, $C_1$ - $C_8$ linear or branched alkyl groups, $C_6$ - $C_{10}$ aryl groups, $C_1$ - $C_8$ linear or branched substituted alkyl groups, and $C_6$ - $C_{10}$ substituted aryl groups.

**13.** An amino acid derivative of Claim 1, comprising an allyl ether-protected tyrosine derivative.

**14.** A compound of Claim 3 wherein the allyl-protected amino acid side-chain is represented by the general formula

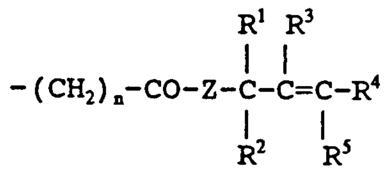

wherein n is 1 or 2; Z is either O or NH; and $R^1$, $R^2$, $R^3$, $R^4$, and $R^5$ are independently selected from the group consisting of hydrogen, $C_1$ - $C_8$ linear or branched alkyl groups, $C_6$ - $C_{10}$ aryl groups, $C_1$ - $C_8$ linear or branched substituted alkyl groups, and $C_6$ - $C_{10}$ substituted aryl groups.

**15.** An amino acid derivative of Claim 1, wherein the amino acid residue is selected from the group consisting of aspartic acid, glutamic acid, asparagine, and glutamine, and the side-chain protecting group is an allyl ester or an allyl amide.

**16.** A compound of Claim 3 wherein the allyl-protected amino acid side-chain is represented by the general formula

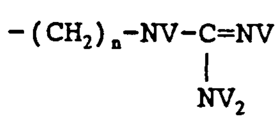

wherein V is independently selected from the group consisting of hydrogen and

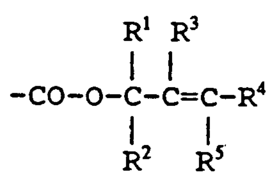

wherein $R^1$, $R^2$, $R^3$, $R^4$, and $R^5$ are independently selected from the group consisting of hydrogen, $C_1$ - $C_8$ linear or branched alkyl groups, $C_6$ - $C_{10}$ aryl groups, $C_1$ - $C_8$ linear or branched substituted alkyl groups, and $C_6$ - $C_{10}$ substituted aryl groups.

**17.** An amino acid derivative of Claim 1, comprising a *mono*-allyloxycarbonyl-protected arginine derivative or a *bis*-allyloxycarbonyl-protected arginine derivative.

**18.** A compound of Claim 3 wherein the allyl-protected amino acid side-chain is represented by the general formula

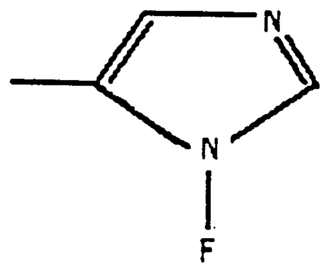

wherein F is

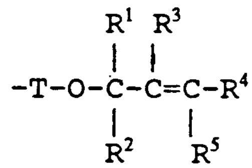

wherein T is a carbonyl group or a methylene group; and $R^1$, $R^2$, $R^3$, $R^4$, and $R^5$ are independently selected from the group consisting of hydrogen, $C_1$ - $C_8$ linear or branched alkyl groups, $C_6$ - $C_{10}$ aryl groups, $C_1$ - $C_8$ linear or branched substituted alkyl groups, and $C_6$ - $C_{10}$ substituted aryl groups.

**19.** An amino acid derivative of Claim 1, wherein the amino acid is histidine and the side-chain protecting group is either an allyloxymethyl group or an allyloxycarbonyl group.

**20.** A solid support for solid-phase peptide synthesis, having bound thereto an $^{\alpha}N$-protected amino acid derivative in which the side-chain thereof is protected with an allyl side-chain protecting group.

**21.** The side-chain protected amino acid derivative of Claim 20 wherein the allyl side-chain protecting group is selected from the group consisting of allyloxycarbonyl, allyloxymethyl, allyl ether, allyl thioether, allyl ester, and allyl amide.

**22.** The solid support of Claim 20, which is a polystyrene graft copolymer resin.

**23.** The solid support of Claim 22, comprising a polyethylene glycol-polystyrene graft copolymer resin.